# FASCICULE DE BREVET EUROPEEN

(11) **EP 2 082 732 B1**
(45) Date de publication et mention de la délivrance du brevet: **16.03.2016**
(21) Numéro de dépôt: 09151260.8
(22) Date de dépôt: 23.01.2009
(51) Int. Cl.: A61K 9/00, A61K 9/20, A61K 31/573

(54) **Comprimés orodispersibles de prednisolone**
Im Mund dispergierbare Tabletten von Prednisolon
Orodispersible tablets of prednisolone

(30) Priorité: 25.01.2008 FR 0850484
(43) Date de publication de la demande: 29.07.2009
(73) Titulaire: Substipharm Developpement, 75016 Paris (FR)
(72) Inventeur: Berthier, César, 75116 Paris (FR); Besse, Jérôme, 33127 Saint Jean d'Illac (FR)
(74) Mandataire: Regimbeau

(56) Documents cités:
- WO-A-99/40898
- WO-A-2005/102271
- WO-A-2005/102287
- WO-A-2007/143061

## Description

L'invention a pour objet des comprimés orodispersibles obtenus par compression directe de formulations pharmaceutiques contenant de la prednisolone, ou un sel d'addition de celle-ci.

La prednisolone, ou 11 bêta, 17 alpha,21-trihydroxyprégna-1,4-diène-3,20-dione, est un corticostéroïde de synthèse, ayant les propriétés pharmacologiques suivantes : anti-inflammatoire, immunosuppresseur et glucocorticoïde. Ses indications thérapeutiques sont particulièrement le traitement des collagénoses, des connectivites, de certains troubles dermatologiques, de certains troubles digestifs, de certains troubles endocriniens, de certains troubles infectieux, de certains troubles néoplasiques, de certains troubles néphrologiques, de certains troubles neurologiques, de certains troubles ophtalmologiques, de certains troubles ORL, de certains troubles respiratoires (asthme), de certains troubles rhumatologiques et en prophylaxie / traitement du rejet de greffe ou de la réaction du greffon contre l'hôte. Les sels d'addition de la prednisolone pouvant être utilisés sont bien connus de l'homme du métier. La prednisolone est le plus fréquemment utilisée sous forme de sel tel que le métasulfobenzoate sodique de prednisolone.

Actuellement, la référence Solupred^{®}, prednisolone sous forme de métasulfobenzoate sodique, est disponible sous la formes de comprimés effervescents, de solutions buvables et de comprimés orodispersibles.

WO2007/143061 décrit des comprimés orodispersibles comprenant une prednisolone revêtue.

WO99/40898 décrit des comprimés sublinguaux comprenant de la prednisolone.

Les comprimés orodispersibles sont des formes pharmaceutiques solides, destinées à l'administration par voie orale, pour une désagrégation rapide dans la bouche avant d'être avalé. L'absorption du principe actif dans une partie quelconque de la cavité buccale et/ou l''action locale du principe actif ne sont pas recherchées. Conformément aux Pharmacopées, notamment à la Pharmacopée Européenne, ils doivent se désagréger dans la bouche en moins de 3 minutes. Ces comprimés orodispersibles doivent de se déliter rapidement en bouche indépendamment de la solubilité du principe actif, d'où l'utilisation d'agent(s) délitant(s) (encore appelés agents désintégrants). Par ailleurs, la formulation doit être telle qu'une fois dispersée ou désintégrée, elle ne confère pas dans la cavité buccale une sensation granuleuse et farineuse, ce qui serait particulièrement inconfortable.

L'invention a pour but principal de proposer un générique du Solupred^{®}, sous forme de comprimé orodispersible. Pour pouvoir bénéficier de l'appellation « générique », les comprimés développés doivent présenter la même composition qualitative et quantitative en principes actifs et la même forme pharmaceutique que la référence Solupred ^{®}.

Le comprimé orodispersible de référence, Solupred^{®}, comprend la prednisolone sous forme de métasulfobenzoate sodique avec les excipients suivants : dispersion de polyacrylate à 30% (Eudragit^{®} NE 30D), silice colloïdale hydrophobe, mannitol (granulé), mannitol (poudre), crospovidone, aspartame et stéarate de magnésium. La dispersion de polyacrylate sert à enrober la prednisolone dans le but de masquer son amertume. Par ailleurs, ces particules enrobées ont une granulométrie contrôlée et une taille de particules plus importante (par rapport à celles non enrobées), ce qui facilite les opérations de mélange des poudres et de compression. La formulation de la référence est donnée dans le tableau 1 suivant:

**Tableau 1**

| **Composants** | **mg/comprimé** |
|---|---|
| Métasulfobenzoate sodique de prednisolone | 31,44 |
| Equivalent prednislone base | 20 |
| Aspartame | 5 |
| Stéarate de magnésium | 1,6 |
| Crospovidone | 12 |
| Eudragit^{®} | 6,29 |
| Silice colloïdale hydrophobe | 1,57 |
| Mannitol (granulé) | 88,42 |
| Mannitol (poudre) | 29,48 |

Le comprimé orodispersible selon l'invention doit en outre pouvoir être obtenu par compression directe.

La compression directe ne comprend pas d'étape de granulation préalable à la compression et permet un gain de temps considérable. Etant donné que la prednisolone ou ses sels d'addition, comme la plupart des principes actifs, possède une mauvaise aptitude à la compressibilité, elle doit être mélangée à des excipients, qui sont directement compressibles et compatibles avec le principe actif, pour pouvoir subir une compression directe.

La compression directe est effectuée sur des machines rotatives à haute vitesse. La trémie d'alimentation fonctionne en général par gravité et est sensible à l'agglomération des poudres ou à leur prise en masse. La rhéologie du mélange de poudres à comprimer est donc un facteur à prendre en considération pour garantir l'uniformité de masse des comprimés et l'uniformité de teneur.

Un autre inconvénient de la technique de compression directe provient du risque de séparation des poudres ou « démélange ». Ce démélange conduit à des comprimés non homogènes en composition.

Ainsi, en utilisant la technique de compression directe, des risques de mauvaise distribution du principe actif dans le mélange à comprimer et/ou de séparation du principe actif et des excipients conduisant à des comprimés non homogènes existent. Par ailleurs, on peut rencontrer des difficultés liées à une mauvaise fluidité du mélange de poudres ou des problèmes de collage, de grippage ou de décalotage, rendant difficile l'éjection du comprimé.

Un excipient, pour être utilisé en compression directe, doit posséder une bonne coulabilité, ne doit pas s'agglomérer de façon spontanée, doit former un comprimé de bonne tenue mécanique ou cohésif sous l'effet d'une force de compression raisonnable et doit permettre un délitement en un temps adapté. De nombreux diluants et liants directement compressibles ont été développés. Certains excipients pour compression directe sont onéreux car ils nécessitent des procédés de préparation élaborés ou l'adjonction de nombreux additifs.

L'homme du métier est toujours à la recherche de formulations alternatives, pour comprimés orodispersibles, qui peuvent être soumises à une compression directe. Par ailleurs, il est désireux de s'émanciper de l'étape préalable d'enrobage de la prednisolone ou l'un de ses sels, ce qui toutefois augmente les risques d'hétérogénéité de la formulation et de démélange. Bien entendu, l'homme du métier souhaite en outre limiter l'utilisation d'excipients à effet notoire. La formulation développée doit permettre également de garantir une faisabilité industrielle.

La formulation selon l'invention permet de résoudre ces problèmes techniques.

L'invention a donc pour objet des comprimés orodispersibles, obtenus par compression directe d'une formulation pharmaceutique appropriée, constitués de prednisolone ou d'un sel d'addition de celle-ci, à titre de principe actif, en une quantité thérapeutique efficace, et d'excipients appropriés.

Dans le cadre de la description de l'invention, on désignera par le terme « prednisolone » aussi bien la prednisolone que l'un quelconque de ses sels d'addition. Lorsque l'on entendra désigner la prednisolone sans inclure ses sels d'addition, on utilisera les termes « prednisolone base ». De même, sauf indication contraire, les termes « principe actif » et « substance active » font référence à la prednisolone base et à l'un quelconque de ses sels d'addition.

La quantité thérapeutique efficace de principe actif est avantageusement telle que la teneur unitaire en prednisolone base soit comprise entre 5 et 20 mg, de préférence la teneur unitaire en prednisolone base est de 5 ou 20 mg. Lorsque le principe actif est un sel d'addition de prednisolone, la teneur en sel dans la formulation est telle que cette dernière comprend de 5 à 20 mg de prednisolone base.

Les excipients sont constitués
- de cellulose microcristalline,
- d'un agent d'écoulement, avantageusement une silice colloïdale hydrophobe,
- d'au moins un arôme et/ou un édulcorant,
- d'un délitant, et
- d'un lubrifiant.

Dans le cadre de la présente invention, on entend désigner par l'expression « comprimé orodispersible » un comprimé non enrobé ou pelliculé qui est destiné à être placé dans la bouche, plus particulièrement sur la langue, où il se disperse avec un temps de désagrégation inférieur à 3 minutes, avantageusement inférieur à 1 minute. Lors de la désagrégation dans la cavité buccale, le principe actif n'est pas absorbé dans une partie quelconque de ladite cavité buccale. Cette définition ne comprend pas les comprimés effervescents, les comprimés à utiliser dans la cavité buccale tels que les comprimés sublinguaux et les comprimés à libération immédiate.

La pharmacopée européenne distingue clairement :
- les comprimés orodispersibles, qui sont des comprimés non enrobés destinés à être placés dans la bouche où ils se dispersent rapidement avant d'être avalé ; et
- les comprimés à utiliser dans la cavité buccale, dont la formulation est établie de façon à permettre une libération lente et une action locale de la substance active, ou la libération et l'absorption de la substance active dans une partie définie de la cavité buccale.

Dans le cadre de la présente invention, la formulation est destinée à la préparation de comprimés par compression directe, ce qui signifie que suite à l'étape de mélange du principe actif avec ses excipients, le mélange obtenu peut directement être comprimé pour donner des comprimés sans étape de granulation préalable.

Par excipient à effet notoire, on entend toute substance dont la présence au sein de la composition nécessite des précautions d'emploi pour certaines catégories de patients.

La formulation selon l'invention permet avantageusement l'utilisation de la prednisolone brute, c'est-à-dire telle quelle, non enrobée, non pelliculée (c'est-à-dire n'ayant pas subi d'étape préalable d'enrobage - tel qu'un revêtement Eudragit^{®}-, de pelliculage et/ou de mise en forme ou granulation telle que l'obtention de microsphères). La prednisolone n'est pas non plus adsorbée ou adhérée à un support pharmaceutiquement acceptable, tel que des particules de sucre.

On utilise de préférence la prednisolone sous forme de métasulfobenzoate sodique de prednisolone.

La granulométrie de ce principe actif (métasulfobenzoate sodique de prednisolone) est centrée autour de 4 à 10 µm (taille moyenne des particules) avec un D(v ; 0,90) < 25 µm (taille de particules pour laquelle 90% en volume passant de l'échantillon se trouve en dessous de cette dimension).

La cellulose microcristalline remplit la fonction de liant. Plusieurs types de cellulose microcristalline sont disponibles. Ils diffèrent par leur mode de fabrication, la taille des particules, l'humidité résiduelle, l'écoulement ou d'autres propriétés physiques. Pour le développement du comprimé orodispersible selon l'invention, la cellulose microcristalline préférée présente une perte à la dessication inférieure à 5% (avantageusement comprise entre 3% et 5%) et une distribution granulométrique adaptée aux autres composants (taille moyenne de particules de 140 à 200 µm). La cellulose microcristalline présente également avantageusement un bon écoulement et une bonne aptitude à la compression directe. Comme exemple de cellulose microcristalline commerciale particulièrement adaptée, on peut notamment citer l'Avicel^{®} PH 200 et la Vivapur^{®} 12. La cellulose microcristalline de nom commercial Vivapur^{®} 12 présente la granulométrique suivante : D(v ; 0,10) < 80 µm, D(v ; 0,50) 140 - 200 µm, et D(v ; 0,90) > 250 µm. Les particules de ces celluloses commerciales se caractérisent par ailleurs une forme cristalline particulière, des bâtonnets, qui présente la propriété de bien s'écraser, ce qui favorise la cohésion.

Par ailleurs, dans la formulation selon l'invention, la cellulose microcristalline joue également le rôle d'un diluant (agent utilisé pour compléter la composition pharmaceutique préparée jusqu'à obtention du volume total prédéterminé contenant la quantité choisie de prednisolone). La teneur en cellulose microcristalline correspond donc la quantité nécessaire pour compléter la composition pharmaceutique préparée jusqu'à obtention du volume total prédéterminé.

La teneur totale en cellulose microcristalline est comprise entre 55% et 80% en masse, plus avantageusement entre 60% et 80% en masse, encore plus avantageusement entre 65% et 80% en masse, par rapport à la masse totale de la formulation.

Cette cellulose microcristalline présente avantageusement les caractéristiques de procédé suivantes : un écoulement rapide (de préférence inférieur à 10 s) et un volume apparent faible (de préférence inférieur à 20 ml).

Sans vouloir être liés à une quelconque interprétation, les inventeurs sont d'avis que la cellulose microcristalline remplit également les fonctions techniques d'un délitant. En particulier, les inventeurs ont constaté qu'il n'était pas nécessaire d'introduire dans la formulation de l'amidon (en particulier du Lycatab® qui est un amidon de maïs prégélatinisé).

En termes de coût du procédé, la cellulose microcristalline est moins chère que le mannitol et plus facile de manipulation, ce qui permet d'augmenter la productivité et de diminuer les coûts.

La formulation comprend un agent d'écoulement garantissant une répartition homogène de la prednisolone de faible granulométrie au sein de la composition pharmaceutique et un bon écoulement du mélange final. Ainsi, l'utilisation d'un agent d'écoulement permet de répartir correctement la substance active dans la formulation.

L'agent d'écoulement représente alors avantageusement de 0,01% à 1% en masse par rapport à la masse totale de la formulation.

Parmi les agents d'écoulement, on peut notamment citer le dioxyde de silice, en particulier la silice colloïdale, le silicate de magnésium ou de calcium, le talc. La silice colloïdale est préférée. Selon une variante avantageuse de l'invention, l'agent d'écoulement est la silice colloïdale anhydre.

Il est possible d'utiliser la silice colloïdale Aerosil^{®} R972 qui, d'une manière avantageuse, présente également de bonnes propriétés hydrophobes (taux d'humidité après 2 heures à 105°C : ≤ 0,05%), ce qui permet au comprimé orodispersible d'être moins sensible aux phénomènes de reprise d'humidité.

La formulation comprend également un délitant (agent désintégrant). Un délitant est une substance dont le rôle est d'accélérer la désagrégation du comprimé, donc sa dispersion dans la cavité buccale. Dans le cadre de la présente invention, tout délitant pourra être utilisé. On peut ainsi citer, comme exemple de délitant, l'acide alginique, le benzoate de sodium, la carboxyméthylcellulose calcique, la carboxyméthylcellulose sodique, la carboxyméthylcellulose sodique réticulée (en anglais : croscarmellose sodium), la crospovidone, le dibéhénate de glycérol, la gomme de guar, le silicate d'aluminium et de magnésium, l'éther méthylique de cellulose (méthylcellulose), la polacrilin-potassium, le glycolate d'amidon de sodium et des dérivés polymères d'acide acrylique.

Selon une variante avantageuse de l'invention, le délitant est choisi dans le groupe constitué par la carboxyméthylcellulose sodique réticulée, le glycolate d'amidon sodique, et la crospovidone.

Dans la formulation, la teneur en délitant est avantageusement comprise entre 2 et 15% en masse, plus avantageusement entre 5% et 15% en masse, encore plus avantageusement comprise entre 8% et 12% en masse, par rapport à la masse totale de la formulation.

Lorsque le délitant est la crospovidone, sa teneur en masse dans la formulation, par rapport à la masse totale de ladite formulation, est avantageusement de 10%. Cette valeur est supérieure aux valeurs classiquement recommandées pour la crospovidone (2 à 5% en masse par rapport à la masse totale de la formulation). Cette teneur plus importante en crospovidone permet, en combinaison avec l'utilisation de la cellulose microcristalline, de s'abstenir d'introduire dans la formulation de l'amidon (en particulier du Lycatab^{®} qui est un amidon de maïs prégélatinisé).

On peut utiliser la crospovidone sous la dénomination Kollidon^{®} CL.

La formulation pharmaceutique selon l'invention comprend au moins un édulcorant et/ou un arôme, qui permet de masquer l'amertume de la prednisolone.

La formulation comprend avantageusement au moins un arôme. Elle peut en outre comprendre un édulcorant, qui accroît le rôle de l'arôme.

En particulier, la formulation selon l'invention comprend, à titre d'arôme, des arômes d'orange-pamplemousse.

Dans la formulation, la teneur en arôme est avantageusement comprise entre 0,5% et 5% en masse, plus avantageusement comprise entre 1% et 3% en masse, par rapport à la masse totale de la formulation.

La formulation selon l'invention comprend avantageusement, à titre d'édulcorant, l'aspartame (son pouvoir sucrant est environ 200 fois plus important que celui du saccharose).

Dans la formulation, la teneur en édulcorant est avantageusement comprise entre 0,1% et 5% en masse, plus avantageusement comprise entre 0,25% et 2% en masse, par rapport à la masse totale de la formulation.

La formulation comprend également un lubrifiant. Un lubrifiant est un excipient qui permet de diminuer le frottement entre les particules dans le comprimé. En outre, le lubrifiant facilite la compression et l'éjection des comprimés. Dans le cadre de la présente invention, tout lubrifiant pourra être utilisé. On peut ainsi citer, comme exemple de lubrifiant, le stéarate de calcium, l'huile végétale hydrogénée de type I, le stéarate de magnésium, l'acide stéarique, le talc, l'acide benzoïque, le stéaryl fumarate, et le stéarate de zinc.

Selon une variante avantageuse de l'invention, le lubrifiant est choisi dans le groupe constitué par le stéarate de magnésium et le stéaryl fumarate.

La teneur en lubrifiant, dans la formulation, est avantageusement comprise entre 0,25% et 5% en masse, par rapport à a masse totale de ladite formulation.

Lorsque le lubrifiant est le stéarate de magnésium, sa teneur en masse dans la formulation, par rapport à la masse totale de ladite formulation, varie avantageusement de 0,5% à 3%, plus avantageusement de 0,5% à 1,5%.

Dans la formulation selon l'invention, les excipients sont constitués exclusivement:
- d'un liant et diluant, la cellulose microcristalline,
- d'un agent d'écoulement, en particulier de la silice colloïdale,
- d'un délitant, en particulier la crospovidone,
- d'un lubrifiant, en particulier du stéarate de magnésium,
- d'arômes et/ou d'édulcorants.

On constate que cette formulation est d'une grande simplicité et qu'elle n'implique pas d'excipients onéreux. Par ailleurs, elle est limitée en excipients à effet notoire. En effet, les excipients utilisés sont tous des excipients classiquement utilisés en formulation. Ils n'imposent pas de conditions particulières de manipulation, qui vont au-delà des connaissances techniques générales d'un ingénieur en formulation.

Selon une variante avantageuse de l'invention, la prednisolone est préalablement mélangée avec l'agent découlement (silice colloïdale) avant ajout des autres excipients.

Lors de la compression directe, aucun phénomène de collage, grippage ou clivage n'est observé. La formulation comprimée se disperse rapidement. Par ailleurs, les comprimés obtenus sont de composition homogène.

Les comprimés obtenus n'ont pas de consistance pâteuse en bouche et se dispersent bien. Il n'y a pas d'arrière goût froid et métallique ni d'amertume résiduelle.

Ces comprimés présentent un temps de désagrégation en bouche inférieur à 3 minutes, avantageusement inférieur 1 minute. Ces comprimés sont, en accord avec les différentes Pharmacopées, en particulier avec la Pharmacopée Européenne, qualifiés de comprimés orodispersibles.

Les comprimés orodispersibles obtenus sont solides, appropriés pour une utilisation par voie orale, d'apparence uniforme, et ont une dureté suffisante pour pouvoir supporter les éventuels chocs lors du transport et du stockage. Les comprimés orodispersibles obtenus ont avantageusement une dureté de 80N±20N. Leur friabilité est avantageusement inférieure à 1%, en accord avec la Pharmacopée Européenne.

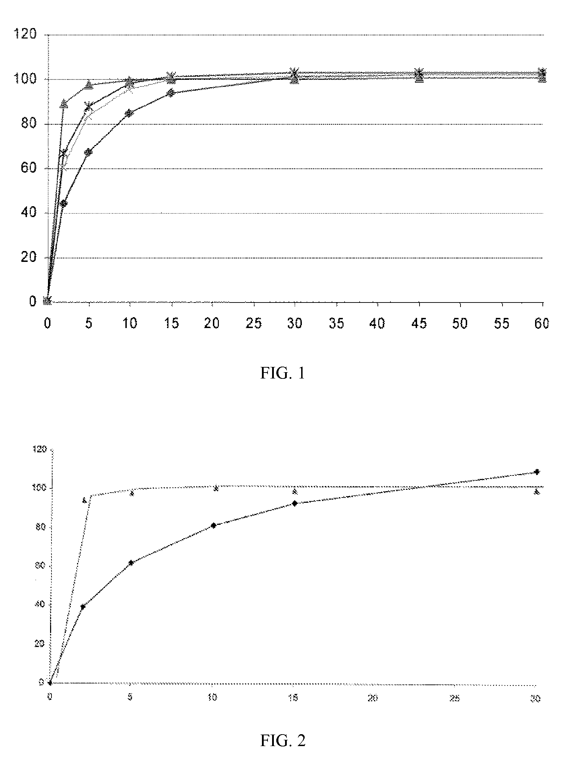

Il a été mis en évidence que la formulation qualitative et quantitative, et son procédé de fabrication, selon l'invention, permet d'obtenir des comprimés orodispersibles présentant un profil de désintégration *in vitro* tel qu'en trois minutes les comprimés orodispersibles sont désagrégés. Par ailleurs, les comprimés orodispersibles permettent l'obtention d'un profil de dissolution *in vitro* de la prednisolone dans une solution NaCl/HCl ajustée à pH 1,2 tel que plus de 75%, avantageusement plus de 80%, encore avantageusement plus de 90%, de la substance active soit libérée en moins de 15 minutes. On constate même que la dissolution du principe actif est quasi-instantanée : l'asymptote est quasi-atteinte en moins de 5 minutes (cf. figures 1 et 2, la figure 2 est un détail de la figure 1).

La formulation selon l'invention permet de fabriquer, par compression directe, des comprimés orodispersibles contenant 20 mg de prednisolone et qui possèdent un profil pharmaco-cinétique *in vivo* suivant :
- de préférence, une aire sous la courbe de concentration pasmatique de 0 à l'infini (AUCO-∞) comprise entre 800 et 2600 ng.h/ml pour la prednisolone et comprise entre 190 et 470 ng.h/ml pour la prednisone,
- de préférence, une valeur de concentration plasmatique maximum (Cmax) comprise entre 10 et 310 ng/ml pour la prednisolone et comprise entre 1 et 50 ng/ml pour la prednisone,
- de préférence, une valeur de Tmax (temps nécessaire à l'obtention de la concentration plasmatique maximum du principe actif) comprise entre 3 et 7 h pour la prednisolone et comprise entre 3 et 7 h pour la prednisone.

Les comprimés orodispersibles selon l'invention sont ensuite conditionnés, avantageusement sous la forme de blisters, afin d'être commercialisés. Les blisters peuvent classiquement être de blisters en aluminium (Alu/Alu). Toutefois, selon une variante avantageuse de l'invention, les blisters sont en complexe Alu-PVC/PCTFE. Tout en présentant des propriétés d'étanchéité similaires, les blisters en complexe Alu-PVC/PCTFE, par rapport à ceux en Alu/Alu, sont plus économiques, alors même que leur coût initial d'achat est supérieur. Cette économie s'explique en partie par le gain en matière observé lorsque l'on utilise le complexe Alu-PVC/PCTFE pour la fabrication des blisters. Pour un blister Alu/Alu, la surface de complexe est de 5184 mm² (48×108 mm). Pour un blister en Alu-PVC/PCTFE supportant la même quantité (10) des mêmes comprimés, la surface de complexe est de 2914 mm² soit une surface inférieure par 44% (2 blisters par boîte). Cette diminution de consommation de matière première compense significativement le surcoût initial du produit (Alu-PVC/PCTFE). Les économies réalisées sont de l'ordre de 20%. On peut également noter que l'utilisation de blisters en Alu-PVC/PCTFE nécessite un investissement moindre en outillage (l'utilisation d'un module de formage à froid pour aluminium n'est pas nécessaire), ce qui se traduit par des économies supplémentaires.

Les exemples qui suivent illustrent l'invention.

### Exemple 1 : Comprimé orodispersible

La formulation suivante a été soumise à une compression directe :

**Tableau 2**

| Composés | Formulation | |
|---|---|---|
| | % | mg/comprimé |
| **Principe actif** | | |
| Métasulfobenzoate sodiquede Prednisolone | 15,72 | 31,44 |
| Equivalent en prednisolone base | | 20,00 |
| **Excipients** | | |
| Cellulose microcristalline¹ | 70,58 | 141,16 |
| Crospovidone² | 10,00 | 20,00 |
| Arôme orange-pamplemousse | 2,00 | 4,00 |
| Stéarate de magnésium³ | 1,00 | 2,00 |
| Aspartame | 0,50 | 1,00 |
| Silice colloïdale hydrophobe⁴ | 0,20 | 0,40 |
| **Total** | 100,00 | 200,00 |

| | | |
|---|---|---|
| ¹ VIVAPUR^{®} 12 commercialisé par la société JRS ² Kollidon^{®} CL commercialisé par la société BASF ³ d'origine végétale, fourni par la société FACI ⁴ Aerosil^{®} R972 commercialisée par la société DEGUSSA | | |

Une étude de compatibilité réalisée par analyse calorimétrique différentielle pour les mélanges binaires suivants :
- cellulose microcristalline (Vivapur^{®} 12) et métasulfobenzoate sodique de prednisolone
- arôme orange-pamplemousse et métasulfobenzoate sodique de prednisolone ne met en évidence aucune interaction potentielle. On peut en conclure que le principe actif est compatible avec ces excipients.

Les comprimés obtenus présentent les propriétés suivantes :
- Dureté : 80N±20N
- Friabilité : conforme Ph.Eur. <1%
- Temps de délitement : conforme Ph.Eur. < 3 min
Dureté : cet essai est destiné à déterminer, dans des conditions définies, la résistance à la rupture des comprimés, mesurée par la force nécessaire pour provoquer leur rupture par compression diamétrale.
Friabilité : cet essai est destiné à déterminer, dans des conditions définies, la friabilité des comprimés non enrobés, c'est-à-dire le phénomène par lequel la surface des comprimés est endommagée ou présente des signes d'abrasion ou de rupture sous l'effet de chocs mécaniques ou d'une attrition.

Le profil de dissolution est représenté sur les figures 1 et 2. Par rapport au produit de référence, Solupred^{®}, le comprimé orodispersible selon l'invention permet une dissolution plus rapide de la prednisolone les 15 premières minutes puis atteint le même plateau.

Figure 1 : comparatif des profils de dissolution *in vitro* pour 3 lots de comprimés orodispersibles Solupred^{®} (◆, ×, ∗) et pour le comprimé selon l'exemple 1(▲), où est reporté le pourcentage de substance active dissoute (par rapport à la quantité initiale comprise dans le comprimé) dans une solution NaCl/HCl ajustée à pH 1,2 en fonction du temps (0-60 minutes).

Figure 2 : comparatif des profils de dissolution *in vitro* pour le comprimé orodispersible Solupred^{®} (◆) et pour le comprimé selon l'exemple 1(▲), où est reporté le pourcentage de substance active dissoute (par rapport à la quantité initiale comprise dans le comprimé) dans une solution NaCl/HCl ajustée à pH 1,2 en fonction du temps (0-30 minutes).

### Exemple 2 : Etude des caractéristiques du mélange final

Les caractéristiques du mélange final sont résumées dans le tableau 3 suivant. Les résultats des essais détaillés ci-dessous ont été obtenus à partir de la fabrication d'un lot de la formulation de l'exemple 1 d'une taille de 500g.

**Tableau 3**

| **ESSAIS** | | **Exemple 1** |
|---|---|---|
| Caractères | | |
| Humidité résiduelle (%)* | | 4,98 |
| Prise d'essai (g) | | 100 |
| Ecoulement (s) | | 6,04 |
| Volume apparent (ml) | V₀ | 236 |
| | V₁₀ | 210 |
| | V₅₀₀ | 189 |
| | V₁₂₅₀ | 186 |
| | V₂₅₀₀ | 184 |
| Aptitude au tassement (ml) | V₁₀-V₅₀₀ | 21 |
| Masse volumique apparente (g.ml-1) | | |
| avant tassement : | m/V₀ | 0,42 |
| après tassement : | m/V₂₅₀₀ | 0,54 |
| Classe granulométrique (µm) | | 125-180 |

| | | |
|---|---|---|
| *L'humidité résiduelle est déterminée grâce à un analyseur électronique de type MA 30 (ou équivalent) sur une prise d'essai de 3g ± 5% à une température de 100°C pendant 15 min. | | |

Les résultats de l'analyse granulométrique du mélange final sont donnés dans le tableau 4 suivant :

**Tableau 4**

| **Ouverture de maille (µm)** | **Refus (%)** | **Refus cumulés (%)** |
|---|---|---|
| 710 | 0,20 | 0,20 |
| 500 | 0,60 | 0,80 |
| 355 | 1,00 | 1,80 |
| 250 | 11,40 | 13,20 |
| 180 | 23,00 | 36,20 |
| 125 | 23,50 | 59,70 |
| 90 | 13,20 | 72,90 |
| Fond | 27,10 | 100,00 |

| | | |
|---|---|---|
| ***Masse retenue** = 100g* | | |

### Conclusion :

Le mélange final obtenu permet d'envisager favorablement la compression. En effet, l'écoulement du mélange se fait en moins de 10 secondes et l'aptitude au tassement est voisine de 20 ml.

### Exemple 3 : Etude des caractéristiques des comprimés

Les résultats des essais détaillés ci-dessous (tableau 5) ont été obtenus à partir de la fabrication d'un lot d'environ 400 comprimés par compression directe à partir de la formulation de l'exemple 1. Le format des poinçons utilisés est plat, rond et de diamètre de 9 mm. Ces poinçons sont caractérisés par la présence d'un chanfrein et d'une barre de confort sur une face.

**Tableau 5**

| **ESSAIS** | **REFERENCE** | **Comprimés selon l'invention** |
|---|---|---|
| Masse moyenne (mg) | - | 199,5 |
| Uniformité de masse | Ph. Eur. (2.9.5) | Conforme |
| Résistance à la rupture (N) | Ph. Eur. (2.9.8) | 79 |
| Désagrégation (s) (eau distillée - 37° C) | Ph. Eur. (2.9.1) | 32 |
| Contrôles dimensionnels (mm) : | | |
| - Epaisseur | - | 2,68 |
| - Diamètre | | 9,07 |
| Humidité (%) *(3g - 100° C - 15 min)* | - | 5,34 |

Les résultats des essais de dissolution de la prednisolone *in vitro* de ces comprimés orodispersibles sont donnés dans le tableau 6 suivant.
Méthode : Spectrophotométrie UV
Volume du milieu de dissolution : 1 000 ml
Longueur d'onde : 237 nm
Vitesse de rotation de l'axe : 75 rpm (axe : palettes tournantes ; lestage : Aucun)

**Tableau 6**

| | **Substance active (%)** | |
|---|---|---|
| **Temps (min)** | **Moyenne** | **CV (%)** |
| 0 | 0 | 0 |
| 2 | 88,69 | 1,83 |
| 5 | 97,13 | 1,87 |
| 10 | 98,94 | 1,88 |
| 15 | 99,27 | 1,92 |
| 30 | 99,67 | 1,99 |
| 45 | 99,78 | 2,00 |
| 60 | 99,87 | 2,03 |

### Conclusion :

D'après la pharmacopée Européenne, le temps de désagrégation d'un comprimé orodispersible, doit être inférieur à 3 minutes. L'objectif fixé était de développer une formule dont le temps de désagrégation soit voisin de 30 secondes : celui-ci est atteint.

De plus, la dissolution de la prednisolone *in vitro* montre un profil très satisfaisant avec des coefficients de variation faibles et une bonne reproductibilité inter comprimés. La production à l'échelle industrielle a donc pu être réalisée.

## Revendications

1. Comprimé orodispersible obtenu par compression directe d'une formulation constituée de prednisolone ou d'un sel d'addition de celle-ci, à titre de principe actif, et d'excipients appropriés, **caractérisé en ce que** dans la formulation les excipients sont constitués exclusivement
- d'un liant et diluant, la cellulose microcristalline,
- d'un agent d'écoulement
- d'un délitant
- d'un lubrifiant
- d'arômes et/ou d'édulcorants
et **en ce que** la teneur en cellulose microcristalline est comprise entre 55% et 80% en masse, par rapport à la masse totale de la formulation.

2. Comprimé orodispersible selon la revendication 1, **caractérisé en ce que** ladite formulation comprend de 5 à 20 mg de prednisolone.

3. Comprimé orodispersible selon les revendications 1 ou 2, **caractérisé en ce que** dans ladite formulation la prednisolone ou l'un des ses sels d'addition est utilisée brute.

4. Comprimé orodispersible selon l'une quelconque des revendications précédentes, **caractérisé en ce que** la cellulose microcristalline présente une perte à la dessiccation inférieure à 5%.

5. Comprimé orodispersible selon l'une quelconque des revendications précédentes, **caractérisé en ce que** le délitant est choisi dans le groupe constitué par la carboxyméthylcellulose sodique réticulée, le glycolate d'amidon sodique, et la crospovidone.

6. Comprimé orodispersible selon l'une quelconque des revendications précédentes, **caractérisé en ce que** l'agent d'écoulement est choisi dans le groupe constitué par le dioxyde de silice, en particulier la silice colloïdale, le silicate de magnésium ou de calcium et le talc, avantageusement la silice colloïdale.

7. Comprimé orodispersible selon l'une quelconque des revendications précédentes, **caractérisé en ce que** le lubrifiant est choisi dans le groupe constitué par le stéarate de magnésium et le stéaryl fumarate.

8. Comprimé orodispersible selon l'une quelconque des revendications précédentes, **caractérisé en ce que** dans ladite formulation la teneur en délitant est comprise entre 2% et 15% en masse, par rapport à la masse totale de la formulation.

9. Comprimé orodispersible selon l'une quelconque des revendications précédentes, **caractérisé en ce que** dans ladite formulation la teneur en agent d'écoulement est comprise entre 0,01% et 1% en masse, par rapport à la masse totale de la formulation.

10. Comprimé orodispersible selon l'une quelconque des revendications précédentes, **caractérisé en ce que** dans ladite formulation la teneur en lubrifiant est comprise entre 0,25% et 5% en masse, par rapport à la masse totale de la formulation.

11. Comprimé orodispersible selon l'une quelconque des revendications précédentes, **caractérisé en ce que** ladite formulation comprend, à titre d'arôme, des arômes d'orange-pamplemousse.

12. Comprimé orodispersible selon l'une quelconque des revendications précédentes, **caractérisé en ce que** ladite formulation comprend, à titre d'édulcorant, l'aspartame.

## Patentansprüche

1. Schmelztablette, die als geeigneter Wirkstoff oder Trägerstoff durch direkte Komprimierung einer Formulierung erhalten wird, die aus Prednisolon oder einem Zusatzsalz desselben gebildet ist, **dadurch gekennzeichnet, dass** in der Formulierung die Trägerstoffe ausschließlich gebildet sind aus
- einem Bindemittel und Verdünnungsmittel, der mikrokristallinen Zellulose,
- einem Ausscheidungswirkstoff
- einem Auslösemittel
- einem Schmierstoff
- Aromen und / oder Süßungsmitteln
und dass der Gehalt an mikrokristalliner Zellulose inbegriffen ist zwischen 55 Masse-% und 80 Masse-% im Verhältnis zur Gesamtmasse der Formulierung.

2. Schmelztablette gemäß Anspruch 1, **dadurch gekennzeichnet, dass** die genannte Formulierung 5 bis 20 mg Prednisolon umfasst.

3. Schmelztablette gemäß Anspruch 1 oder 2, **dadurch gekennzeichnet, dass** in der Formulierung das Prednisolon oder eines seiner Zusatzsalze roh verwendet wird

4. Schmelztablette gemäß einem der voranstehenden Ansprüche, **dadurch gekennzeichnet, dass** die mikrokristalline Zellulose einen Verlust bei der Trocknung von weniger als 5 % aufweist.

5. Schmelztablette gemäß einem der voranstehenden Ansprüche, **dadurch gekennzeichnet, dass** das Auslösemittel aus der Gruppe ausgewählt ist, die durch natriumhaltige, vernetzte Carboxymethylzellulose, natriumhaltiges Stärkeglykolat und Crospovidon gebildet ist.

6. Schmelztablette gemäß einem der voranstehenden Ansprüche, **dadurch gekennzeichnet, dass** der Ausscheidungswirkstoff ausgewählt ist aus der Gruppe, die durch Kieselsäuredioxid, insbesondere kolloidale Kieselsäure, Magnesium- oder Kalziumsilikat und Talkum, vorteilhaft kolloidale Kieselsäure gebildet ist.

7. Schmelztablette gemäß einem der voranstehenden Ansprüche, **dadurch gekennzeichnet, dass** der Schmierstoff ausgewählt ist in der Gruppe, die durch Magnesiumstearat und Fumaratstearyl gebildet ist.

8. Schmelztablette gemäß einem der voranstehenden Ansprüche, **dadurch gekennzeichnet, dass** in der genannten Formulierung der Gehalt an Auslösemittel zwischen 2 Masse-% und 15 Masse-% im Verhältnis zur Gesamtmasse der Formulierung inbegriffen ist.

9. Schmelztablette gemäß einem der voranstehenden Ansprüche, **dadurch gekennzeichnet, dass** in der genannten Formulierung der Gehalt an Ausscheidungswirkstoff zwischen 0,01 Masse-% und 1 Masse-% im Verhältnis zur Gesamtmasse der Formulierung inbegriffen ist.

10. Schmelztablette gemäß einem der voranstehenden Ansprüche, **dadurch gekennzeichnet, dass** in der genannten Formulierung der Gehalt an Schmierstoff zwischen 0,25 Masse-% und 5 Masse-% im Verhältnis zur Gesamtmasse der Formulierung inbegriffen ist.

11. Schmelztablette gemäß einem der voranstehenden Ansprüche, **dadurch gekennzeichnet, dass** die genannte Formulierung als Aroma Orangen- / Pampelmusenaromen umfasst.

12. Schmelztablette gemäß einem der voranstehenden Ansprüche, **dadurch gekennzeichnet, dass** die genannte Formulierung als Süßungsmittel Aspartam umfasst.

## Claims

1. Orodispersible tablet obtained by direct compression of a formulation constituted of prednisolone or an addition salt thereof, as active ingredient, and appropriate excipients, **characterised in that** in the formulation the excipients are exclusively constituted
- of a binder and diluent, microcrystalline cellulose,
- of a flow agent
- of a disintegrator
- of a lubricant
- of flavourings and/or sweeteners
and **in that** the microcrystalline cellulose content is comprised between 55% and 80% by weight, with respect to the total weight of the formulation.

2. Orodispersible tablet according to claim 1, **characterised in that** said formulation comprises from 5 to 20 mg of prednisolone.

3. Orodispersible tablet according to claims 1 or 2, **characterised in that** in said formulation the prednisolone or an addition salt thereof is used crude.

4. Orodispersible tablet according to any of the preceding claims, **characterised in that** the microcrystalline cellulose has a loss on drying less than 5%.

5. Orodispersible tablet according to any of the preceding claims, **characterised in that** the disintegrator is selected from the group constituted of cross-linked sodium carboxymethylcellulose, sodium starch glycolate, and crospovidone.

6. Orodispersible tablet according to any of the preceding claims, **characterised in that** the flow agent is selected from the group constituted of silicon dioxide, in particular colloidal silica, magnesium or calcium silicate and talc, advantageously colloidal silica.

7. Orodispersible tablet according to any of the preceding claims, **characterised in that** the lubricant is selected from the group constituted of magnesium stearate and stearyl fumarate.

8. Orodispersible tablet according to any of the preceding claims, **characterised in that** in said formulation the disintegrator content is comprised between 2% and 15% by weight, with respect to the total weight of the formulation.

9. Orodispersible tablet according to any of the preceding claims, **characterised in that** in said formulation the flow agent content is comprised between 0.01% and 1% by weight, with respect to the total weight of the formulation.

10. Orodispersible tablet according to any of the preceding claims, **characterised in that** in said formulation the lubricant content is comprised between 0.25% and 5% by weight, with respect to the total weight of the formulation.

11. Orodispersible tablet according to any of the preceding claims, **characterised in that** said formulation comprises, as flavouring, orange-grapefruit flavourings.

12. Orodispersible tablet according to any of the preceding claims, **characterised in that** said formulation comprises, as sweetener, aspartame.
